# EUROPEAN PATENT SPECIFICATION

(11) **EP 3 199 643 B1**
(45) Date of publication and mention of the grant of the patent: **08.05.2019**
(21) Application number: 16207282.1
(22) Date of filing: 29.12.2016
(51) Int. Cl.: C12Q 1/6888

(54) **METHOD OF IDENTIFICATION OF EUROPEAN FRESHWATER FISH AND HYBRIDES IN BIOLOGICAL MATERIALS BY S7ICAPS METHOD**
VERFAHREN ZUR IDENTIFIZIERUNG VON EUROPÄISCHEM SÜSSWASSERFISCH UND HYBRIDEN IN BIOLOGISCHEN MATERIALIEN MITTELS S7ICAPS-VERFAHREN
PROCÉDÉ D'IDENTIFICATION DE POISSONS D'EAU DOUCE EUROPÉENS ET D'HYBRIDES DANS DES MATIÈRES BIOLOGIQUES PAR PROCÉDÉ S7ICAPS

(30) Priority: 31.12.2015 CZ 20150957
(43) Date of publication of application: 02.08.2017
(73) Proprietor: Ustav biologie obratlovcu AV CR, v.v.i., 60300 Brno (CZ)
(72) Inventor: MENDEL, Jan, 63600 Brno (CZ); HALACKA, Karel, 60200 Brno (CZ); VETESNIK, Lukas, 63400 Brno (CZ)
(74) Representative: Lunzarová, Lucie

(56) References cited:
- MENDEL JAN ET AL: "Molecular phylogeny of the genusGobioCuvier, 1816 (Teleostei: Cyprinidae) and its contribution to taxonomy", MOLECULAR PHYLOGENETICS AND EVOLUTION, vol. 47, no. 3, 14 March 2008 (2008-03-14) , pages 1061-1075, XP029239272, ISSN: 1055-7903, DOI: 10.1016/J.YMPEV.2008.03.005
- Z. LAJBNER ET AL: "PCR-RFLP assays to distinguish the Western and Eastern phylogroups in wild and cultured tench Tinca tinca : MOLECULAR DIAGNOSTICS AND DNA TAXONOMY", MOLECULAR ECOLOGY RESOURCES, vol. 11, no. 2, 1 March 2011 (2011-03-01), pages 374-377, XP055379724, UK ISSN: 1755-098X, DOI: 10.1111/j.1755-0998.2010.02914.x
- T. THIEL: "SNP2CAPS: a SNP and INDEL analysis tool for CAPS marker development", NUCLEIC ACIDS RESEARCH, vol. 32, no. 1, 2 January 2004 (2004-01-02), pages 5e-5, XP055379613, DOI: 10.1093/nar/gnh006
- SEINEN CHOW ET AL: "Universal PCR primers for S7 ribosomal protein gene introns in fish", MOLECULAR ECOLOGY., vol. 7, no. 9, 1 September 1998 (1998-09-01), pages 1247-1263, XP055380165, GB ISSN: 0962-1083, DOI: 10.1046/j.1365-294x.1998.00406.x

## Description

### Field of the invention

The present invention relates to a method of identification of European freshwater fish and their hybrids in biological materials and fish products using S7iCAPS method and further relates to a novel primer design, diagnostic JGM regions and their unique lengths, specific restriction patterns and identification kits.

### Background of the invention

Due to the growing global effort to map and protect the real diversity of freshwater fish, including defining their native areas, there is a need to improve current identification methods or develop completely new ones. Knowledge and understanding of real biodiversity and its protection is an important subject of international and national policies in most states. Definite identification of fish is important to protect the original fish gene pool in each state and to set the proper management. An important group of fish species are the protected species which, based on the European Union regulations (Council Directive 92/43/EEC), require different protection regimen and for which protected European Union areas have been delimited - Natura 2000 and EMERALD network of the Bern Convention, with permanent periodic monitoring. In case of a threat of disappearance of populations of one of the "critically endangered" species, a rescue program is initiated, in which the genetic identification is the key matter. The Ministry of Agriculture of the Czech Republic has implemented a program to support the "Introductions of particularly endangered species into the wild", but in most cases still without respecting the protection of genetic diversity.

Definite identification of fish is important also in terms of relatively frequent hybridization. In the literature, there have been described hybridization events in > 95 fish families (Schwartz, F. J. 1981. World literature to fish hybrids with an analysis by family, species, and hybrid: Supplement 1. NOAA Technical Report NMFS SSRF-750, 507 p.; Schwartz, F. J. 2001. Freshwater and marine fish family hybrids: A worldwide changing scene revealed by the scientific literature. Journal of the Elisha Mitchell Scientific Society, 117 (1), 62-65; Scribner, K. T., Page, K. D., Bartron, M. L. 2001. Hybridization in freshwater fishes: a review of case studies and cytonuclear methods of biological inference. Reviews in Fish Biology and Fisheries 10, 293-323; Bartley D. M., Rana K., Immink A. J. 2001. The use of inter-specific hybrids in aquaculture and fisheries. Reviews in Fish Biology and Fisheries 10, 325-337). For some production fish the hybridization is directly required (whitefishes, charrs, etc.). For others, the hybridization is undesirable, which also applies to imports and the introduction of non-native lineages and species for the particular geographic area. Without a timely genetic identification, the genetic hybridizations may be one of the causes of the destruction of the original intraspecific and interspecific diversity.

A reliable genetic determination of individual lineages and breeds is also necessary in intensive farming to preserve the breed purity, to increase and to improve production efficiency, for the preservation of resistance to diseases and various stress factors and to set the proper breeding management in general. Since 1996, the Ministry of Agriculture of the Czech Republic operates a "National program for conservation and utilization of animal genetic resources" and financially supports registered breeders in attending to the conservation of genetic resources of livestock and other farm animals, including fish.

Also the prevention of falsification of fish products, eggs, fry and young fish is considerable, the falsification leading to specific mix-ups with different quality and price. European Union Council Regulation 104/2000 contains a duty to indicate also the specific name, geographic origin, etc. in the fish products labelling.

In the world, there are known several methods and procedures for species/lineages determination of fish. Methods based on the morphological identification are used for species determination of whole or mildly modified fish (Kottelat, M., Freyhof, J. 2007. Handbook of European freshwater fishes. Kottelat, Cornol, Switzerland and Freyhof, Berlin, Germany). However, in case of the loss or absence of external characters of determination, this method is difficult to be applied. Additionally, this method belongs to highly invasive methods. Methods based on an analysis of proteins extracted from muscle, liver, etc. are also used for identification of fish, such as allozyme analysis (Lusková, V., Šlechtová, V., Povž, M., Šlechta, V., Lusk, S. 1997. Genetic variability of Chondrostoma nasus population in rivers of the Black Sea and the Baltic Sea drainage systems. Folia Zoologica 46 (Suppl. 1), 27-36), isoelectric focusing (Ataman, C., Celik, U., Rehbein, H., 2006. Identification of some Aegean Fish by native isoelectric focusing. European Food Research and Technology, 222, 99-104) or immunological techniques (Dominquez, E., Perez, M. D., Puyol, P., Calvo, M. 1997. Use of imunological techniques for detecting species substitution in raw and smoked fish. Zeitschrift für Lebensmitteluntersuchung und -Forschung A, 204/4, 279-281). Their main limitation is a rapid protein degradation at higher temperatures, a high risk of mutual reactivity or problems associated with the tissue-specificity, a very limited level of polymorphism, a higher rate of subjective evaluation and a highly invasive character.

Currently, for the determination of a wide range of fish, molecular genetic methods based on a DNA analysis are more frequently used. These methods have many advantages when compared to the methods using the protein analysis. DNA is present in all animal tissues, in most cases it doesn't depend on the source of the sample and it can be isolated from all life stages of the organism. DNA degrades more slowly and even a small amount is sufficient because of PCR method. In most cases, the non-invasive methodology, strong informativeness and the possibility to choose a co-dominant marker are crucial.

Due to its accuracy, the classical Sanger sequencing presents a significant contribution to the identification studies of European fish. This method is often used for validation of the results obtained by other methods. Various nuclear markers with diverse degrees of variability are used for fish species identification. For exon-sequencing for example RAG1 (recombination activating gene 1), large ribosomal subunit 28S and rhodopsin are used (Choleva, M., Musilova, Z., Kohoutova-Sediva, A., Paces, J., Rab, P., Janko, K., 2014. Distinguishing between Incomplete Lineage Sorting and Genomic Introgressions: Complete Fixation of Allospecific Mitochondrial DNA in a Sexually Reproducing Fish (Cobitis; Teleostei), despite Clonal Reproduction of Hybrids. PLoS ONE 9(6): e80641). Automated sequencing of the nuclear coding regions often exhibits a reduced variability and therefore a low determination ability (Li Ch., Ortí G., Zhang G., Lu G. 2007. A practical approach to phylogenomics: the phylogeny of ray-finned fish (Actinopterygii) as a case study. BMC Evolutionary Biology 7, 44). For intron-sequencing, for example the intron of the beta subunit of adenosine triphosphate synthase gene (Atp-β) and the second intron of the actin gene (Act-2) are used (Touriya A, M. Rami, G. Cattaneo-Berrebi, C. Ibanez, S. Augros, E. Boissin, A. Dakkak, and P. Berrebi. 2003. Primers for EPIC amplification of intron sequences for fish and other vertebrate population genetic studies. BioTechniques 35: 676-682). Also the first or second intron of ribosomal S7 r-protein (RPS7) can be used (Perea, S., Böhme, M., Zupančič, P., Freyhof, J., Šanda, R., Özuluǧ, M., Abdoli, A., Doadrio, I. 2010. Phylogenetic relationships and biogeographical patterns in Circum-Mediterranean subfamily Leuciscinae (Teleostei, Cyprinidae) inferred from both mitochondrial and nuclear data. BMC Evolutionary Biology, 10, 265). S7 r-protein is one of the proteins playing an important role in the template and 40S subunit interaction (Mundus, D. A., Bulygin, K. N., Ven'yaminova, A. G., Vladimirov, S. N., Vratskikh, L. V., Repkova, M. N., Yamkovoi, V. S., Karpova, G. G. 1993. Human placenta 40S ribosomal subunit affinity modification by oligoribonucleotide derivatives containing the AUG codon. Ribosomal proteins composing the codon-anticodon interaction site. Molecular Biology, 27, 91-95). Nuclear marker RPS7 consists of 7 exons and 6 introns and it was found out that for some freshwater fish species, the first intron architecture is significantly diagnostic (Annilo, T., Laan, M., Stahl, J., Metspalu, A. 1995. The human ribosomal protein S7-encoding gene: Isolation, structure and localization in 2p25. Gene, 165, 297-302; Mendel, J., Lusk, S., Vasileva, E. D. et al. 2008. Molecular phylogeny of the genus Gobio Cuvier, 1816 (Teleostei: Cyprinidae) and its contribution to taxonomy. Mol. Phylogenet. Evol., 47, 1061-1075). PCR primer pairs for amplification of this marker were described in the publication Chow, S., Hazama, K. 1998. Universal PCR primers for S7 ribosomal protein gene introns in fish. Molecular ekology, 7, 1255-1256. But these primer sets very often provided unsatisfactory results, which were understandable because the authors have proposed a primer design only on three fish taxa (Oncorhynchus keta, Thunnus spp., Fugu rubripes), genetically and geographically very divergent from species of European freshwater ichthyofauna. Moreover, the frequent presence of indels causing a frameshift, the missing platform of reference homozygous sequences and in general the marker fragmentation in genetic databases causes significant difficulties in the use of intron markers.

Sequencing technique - DNA barcoding, using the mitochondrial marker COI (cytochrome c oxidase I) as common identification barcode, has already created informational database platform (BoLD) and standardized methodology. It also satisfactorily solves the problem of marker fragmentation and complicated comparison. However, it has its major limitations - it cannot identify hybrids correctly and offers an insufficient one-genome (maternal) view of the object to be examined, because it is the nuclear genes that are primarily responsible for the phenotype.

Fingerprint techniques, as PCR-RFLP (restriction fragment length polymorphism) and AFLP (amplified fragment length polymorphism), are also useful in studies of determination of some freshwater fish species and their hybrids (Campbell, D., Bernatchez, L. 2004. Generic scan using AFLP markers as a means to assess the role of directional selection in the divergence of sympatric whitefish ecotypes. Mol. Biol. Evol 21 (5), 945-956). STR analysis is used especially in population biology, when estimating population parameters, paternity studies, etc. (Baker, A. J. 2000. Molecular methods in ecology. Blackwell science Ltd., Oxford), but due to the high sensitivity it is used in identification studies as a second step technique, usually after DNA sequencing. Furthermore, the high specificity of the primers prevents an easy use in determination studies of large groups of different taxonomic levels.

SPInDel concept uses insertions and deletions for species identification of the different groups of eukaryotes, including some fish species. However, this approach is limited by its design, which is based solely on sequence variability of mitochondrial rRNA genes (Carneiro J., Pereira F., Amorium A. 2012. SPInDel: a multifunctional workbench for species identification using insertion/deletion variants. Molecular Ecology Resources 12, 1190-1195; Pereira F., Carneiro J., Matthiesen R., Van Asch B., Pinto N., Gusmao L., Amorim A. 2010. Identification of species by multiplex analysis of variable-length sequences. Nucleic Acids Research 38, e203).

Currently, second-generation sequencing techniques are emerging ("next-generation sequencing", NGS) on different platforms (pyrosequencing, sequencing technologies Illumina, SOLID, Ion Torrent, etc.). These approaches, however, require a demanding hardware and software for processing huge amounts of data. They focus primarily on the completion of entire genomes. They represent a large investment, expensive operation and expensive reagents.

Other molecular approaches for animal species identification utilize techniques of biochips (Cheng J., Sheldon E. L., Wu, L., Uribe, A., Gerrue, L. O., Heller, M. J. and O'Connell, J. P. 1998, Preparation and hybridization analysis of DNA/RNA from E. coli on microfabricated bioelectronic chips. Nature Biotechnology 16: 541-546). This technique, however, is primarily associated with commercial production and distribution of cosmetic products, food and feed for consumption by humans and animals. It is used for verification of the presence/absence of animal ingredients in the mixture without species specification. The method of real-time PCR for detection and quantification of a few fish species is of a similar character.

### Summary of the invention

Disadvantages of European freshwater fish determinations from biological materials and fish products by methods based on the identification of proteins, by direct sequencing and genotypization of mitochondrial genes and by the latest qualitative and quantitative techniques are eliminated by the method of identification of freshwater fish in biological materials and fish products by S7iCAPS method (S7 intron cleaved amplified polymorphic sequence), in accordance with present invention, which is characterized in that the identification of European ichthyofauna taxa and hybrids thereof is carried out by conventional PCR with species-specific or group-specific primer pairs which define a partial species/lineages unique nuclear sequence of the first intron of S7 gene, called Johann Gregor Mendel identification region (JGM region). The sequences of individual primers are shown in Table 1, wherein the obtained amplicons, in the case of genera with more species/lineages, are either sufficiently length polymorphic (due to the presence of indels; Table 2) and/or are further digested by specific restriction endonucleases (Table 3), in which CAPS design is used, where the restriction sites are located in the species/lineages unique indel positions (InDel-CAPS marker) and/or in specific point mutations - SNPs (single nucleotide polymorphism, SNP-CAPS marker), as shown in Table 2.

**Table 1. Primer design and the specific Ta temperature in the identified genera and species**

| Family | Genus | Species and lineage | Primer abbreviation | Sequence 5' → 3' | Annealing temperature (°C) | Approximate length^{a} of the JGM region (bp) |
|---|---|---|---|---|---|---|
| Acipenseridae | *Acipenser, Huso* | *A. baeri, A. ruthenus, A. stellatus, A. gueldenstaedtii, A. sturio, H. huso* | AcS7F | TGGYDTTTCCCGTACTGTR | 60 | 440 |
| | | | AcS7R | CTGGTRCTGAACATGGCCTAT | | |
| Nemacheilidae | *Barbatula* | *B. barbatula* - 3 lineages | BaS7F | TGCCCAGCTATAAGGA | 60 | 435 |
| | | | BaS7R | TTTATCTTAATMGTCGATGAG | | |
| Centrarchidae | | *L. gibbosus, M. salmoides, M. dolomieu* | CeS7F | GTTGTTTAATRTTTGCGTTTG | 60 | 660 |
| | *Lepomis, Micropterus* | | CeS7R | GGTACTGAACATGGCCTRTG | | |
| Cobitidae | *Cobitis* | | CoS7F | TKGTTKTCCTCATGTAYTGGR | 60 | 515 |
| | | *C. elongatoides, C. tanaitica, C. taenia, C. taurica, C. strumicae, C. fahirae, C. melanoleuca, C. choii, C. vardarensis, C. lutheri, C. paludica* | CoS7R | GWATCACAACAGAAACCACG | | |
| | *Sabanejewia* | *S. balcanica* | SabS7F | TGAATGTGCCCAGCTAGAGG | 60 | 550 |
| | | | SabS7R | ACTGAAATAATATGCCGTTTG | | |
| | *Misgurnus* | *M. fossilis, M. nikolskyi* | MiS7F | TGTGAGTATRTTTGRCRATG | 60 | 475 |
| | | | MiS7R | AGAGTTTAAMGCTCGYACCTT | | |
| Cottidae | *Cottus* | *C. gobio, C. poecilopus* - *2 lineages*, C. microstomus* | CoS7F | GTTTAATCTTYGCGTGGACCC | 60* a 64 | 420 |
| | | | CoS7R | TGAACATGGCCGTTGTGTC | | |

| Family | Genus | Species and lineage | Primer abbreviation | Sequence 5'→ 3' | Annealing temperature (°C) | Approximate length^{a} of the JGM region (bp) |
|---|---|---|---|---|---|---|
| Cyprinidae | | | CyS7F | TTCACTGAGATGCGTTAGATG | 60 | 470 |
| | *Abramis, Rutilus, Scardinius, Blicca, Ballerus, Leuciscus, Leuciscus, Squalius, Chondrostoma, Alburnus, Vimba* | | CyS7R | GACAYACAAWCTGATKYGTCT | | |
| | | *A. brama, B. bjoerkna, S. erythrophthalmus, S. plotizza, S. acarnanicus, S. hesperidicus, L. aspius, B. ballerus, B. sapa, L. leuciscus, L. idus, genus Rutilus* - *R. ohridanus, R. prespensis, R. aula, R. basak, R. pigus, R. virgo, R. ylikiensis, R. panosi, R. frisii, R. caspicus, R. rubilio, R. rutilus, R. heckelii R. meidingeri, R. karamani, R. sp. Sperchios, genus Squalius* - *S. cephalus* - *2 lineages, S. sp. SP-2010a, S. pyrenaicus, S. keadicus, S. castellanus, S. prespensis, S. valentinus, S. torgalensis, S. squallus, S. orientalis, S. malacitanus, S. illyricus, S. carolitertii, genus Chondrostoma* - *Ch. nasus, Ch. oxyrhynchum, Ch. miegii, Ch. turiense, Ch. angorense, Ch. lemmingii, Ch. oretanum, Ch. arcasii, Ch. knerii, Ch. willkommii, Ch. occidentale, Ch. phoxinus, Ch. polylepis, Ch. duriense, genus Alburnus - A. alburnus* - *3 lineages, A. filippii, A. belvica, A. arborella, A. sp. SP-2010, A. kotschyi, A. escherichii, V. vimba, V. melanops* | | | | |
| | *Alburnoides* | | AlS7F | TTCRCTGAGATGSGTTAGATG | 60 | 455 |
| | | *A. bipunctatus, A. tzanevi, A. rossicus, A. sp. 1 (Danube 1), A. sp. 2 (Danube 2), A. sp. 3 (Greece, A. thessalicus), A. economoui (Greece, Sperchios), A. sp. 5 (Azerbaijan, Tugcay), A. maculatus, A.ohridanus, A. prespensis complex (A. devolli, A. fangfangae, A. prespensis), A. strymonicus, A. fasciatus, A.kubanicus, A. eichwaldii* | AlS7R | GACATAMAATYTGWTGCGTCT | | |
| | *Cyprinus, Carassius* | *C. carpio, C. carassius, C. gibelio, C. langsdorfii* | CaS7F | ATTYAGACAGGGATGCGTTAG | 60 | 590 |
| | | | CaS7R | AAGCGTCAGCASTAACATCST | | |
| | *Leucaspius* | *L. delineatus* - 2 lineages | LeuS7F | TACCTCCATGCATGAGCTTTA | 60 | 850 |
| | | | LeuS7R | TTCGCACTGGTACTGAACAT | | |
| | *Leuciscus, Leuciscus, Alburnus, Squalius, Vimba* | | LeS7F | TTCACTGAGATGCGTTAGATG | 60 | 835 |
| | | *L. leuciscus, L. idus, L. cf. latus SP-2010, L. aspius, genus Alburnus* - *A. alburnus* - *3 linie, A. filippii, A. belvica, A. arborella, A. sp. SP-2010, A. kotschyi, A. escherichii, V. vimba, V. melanops* | LeS7R | TATTTTCGCACTGGTACTGAAC | | |
| | *Hypophthalmichthys, Ctenopharyngodon* | *C. idella, H. molitrix, H. nobilis* | HyS7F | GCCCATGTGCTGTTCTAAGA | 60 | 480 |
| | | | HyS7R | AAAMTGAYAGCCTCAATCTCC | | |
| | *Pelecus* | *P. cultratus* | PeS7F | CCGAAAATGCCTCTATT | 60 | 670 |
| | | | PeS7R | CTATTTTCGCACTGGTACTGA | | |
| | *Pseudorasbora* | *P. parva* - 2 lineages | PsS7F | GCTAACSGCATGCTAAGA | 60 | 590 |
| | | | PsS7R | CGCGCTGGTACTGAAC | | |
| | *Phoxinus* | *P. phoxinus* - 2 lineages, *P. lumaireul* | PhS7F | GAGATRCVTTAGATGCGARTA | 60 | 465 |
| | | | PhS7R | ACATACAATCTGATG CGTCT | | |
| | *Romanogobio* | | RoS7F | AAAATCMCTWCACAGYAAGGC | 60 | 810 |
| | | *R. albipinnatus, R. vladykovi, R. belingi, R. uranoscopus, R. kesslerii, R. banaticus, R. parvus, R. pentatrichus, R. sp. - Albánie, R. elimeius* | RoS7R | TTGACCTCCACGCATGAG | | |
| | *Tinca* | *T. tinca* - 2 lineages, carp x tench | TiS7F | GGCCCATGTGCTGTTCTAAGA | 64 | 660 |
| | | | TiS7R | TTTCGCGCTGGTACTGA | | |
| | *Vimba* | *V. vimba* - 2 lineages, *V. melanops* | ViS7F | CGAAAATGCCTCTATTAAGT | 60 | 595 |
| | | | ViS7R | GATTGTCTCATTTACTAACCC | | |
| Esocidae | *Esox* | *E. lucius* | EsS7F | GCTCTCACTGATCGGAATATG | 60 | 420 |
| | | | EsS7R | CAACTACTGTAGCAGCGTTAG | | |
| Gasterosteidae | *Gasterosteus* | *G. aculeatus* | GaS7F | TAAACCCTTCGTAACGCTTCT | 60 | 500 |
| | | | GaS7R | GCATGTACTGTGGGCTAAGAG | | |
| Gobiidae | | | GobidS7F | GCTATTTCTGCATCGGAACCC | 57 | 475 |
| | | *N. melanostomus, B. gymnotrachelus, N. eurycephalus, N. fluviatilis, N. caspius, N. cephalargoides, N. gorlap, N. platyrostris, N. syrman, N. rhodioni, N. cyrius, N. constructor, N. ratan, P. kessleri, P. semilunaris* - *haploskupina Serbia, haploskupina Ukraine - Dniester, haploskupina Ukrajina- Krym, haploskupina Ukraine* - *Odessa, P. cf. semipellucidus, P. sp. CAS-2008, P. marmoratus* | GobidS7R | CCTAGTTATGCMBSTCT | | |
| | *Babka, Neogobius, Ponticola, Proterorhinus* | | | | | |
| Lotidae | *Lota* | *L. lota* | LoS7F | AACAAACATTTAATCGCCATA | 65 | 475 |
| | | | LoS7R | CGACAGAAAGCCGACCAC | | |
| Percidae | *Gymnocephalus* | *G. cernua* - 3 lineages, *G. schraetser*, G. baloni* - 2 lineages | GyS7F | GTCGTWTGTGTAGCGTGGATG | 58* a 60 | 605 |
| | | | GyS7R | AAAAGACATGTGGGGTTAC | | |
| | *Sander, Perca* | *S. lucioperca, S. volgensis, S. marinus, S. vitreus, P. fluviatilis, P. flavescens* | PeS7F | CTACCGTATACATYGTAAGGC | 60 | 480 |
| | | | PeS7R | GGCGCTGGTACTGAAC | | |
| | *Zingel* | *Z. zingel, Z. streber* | ZiS7F | CCGTCGTTGACAGAGGTTMG | 60 | 440 |
| | | | ZiS7R | GCATGTACATTGGCTAGGCTA | | |
| Salmonidae | *Salvelinus, Salmo, Oncorhynchus* | *S. alpinus, S. fontinalis, S. salar, S. trutta, O. mykiss* | SaS7F | TGGATGRMCRTTGTGTAATKA | 60 | 560 |
| | | | SaS7R | YCGAGTGGTCCATGCCGACT | | |
| | *Salvelinus, Hucho* | *S. alpinus, S. fontinalis, H. hucho* | HuS7F | TGGATGGSCGTTGTGTAATGA | 60 | 560 |
| | | | HuS7R | CMRAGTGGTCCATGCCGACT | | |
| | *Salvelinus* | *S.alpinus, S. fontinalis, S. fontinalis x S. alpinus* | SalS7F | TGGATGGCCGTTGTGTAATGA | 60 | 560 |
| | | | SalS7R | CCGAGTGGTCCATGCCGACT | | |
| Siluridae | *Silurus* | *S. glanis* | SiS7F | CCTCGAGCCGAAAGCGTC | 60 | 445 |
| | | | SiS7R | TTGTGCACTACAGCGGTATGG | | |
| Umbridae | *Umbra* | *U. krameri* | UmS7F | CATGCGTTCCTGTGCCTATCC | 60 | 490 |
| | | | UmS7R | CTGAACATGGCCAACGTCTCT | | |

| | | | | | | |
|---|---|---|---|---|---|---|
| ^{a}Approximate length = rounded and averaged value | | | | | | |

**Table 2. S7iCAPS identification key - the exact length of the JGM region, specific Indel-CAPS and SNP-CAPS markers for selected species/lineages and heterozygotes and hybrids thereof**

| Species/lineage/heterozygote/hybrid | Unique JGM region | CAPS marker 1 | Length of the fragment | CAPS marker 2 | Length of the fragment | CAPS marker 3 | Length of the fragment | CAPS marker 4 | Length of the fragment |
|---|---|---|---|---|---|---|---|---|---|
| | (bp) | | | | | | | | |
| Salvelinus alpinus | **524** | TasI | **243,** 175, 86,20 | x | x | x | x | x | x |
| Salvelinus fontinalis_lineage A | **583** | TasI | **305,** 172, 86, 20 | x | x | x | x | x | x |
| Salvelinus fontinalis_lineage B | **589** | TasI | **394,** 175, 20 | x | x | x | x | x | x |
| S. fontinalis_A/S. fontinalis_B | **583/589** | TasI | **305/394** | x | x | x | x | x | x |
| S. fontinalis_A/S. alpinus | **583/524** | TasI | **243/305** | x | x | x | x | x | x |
| S. fontinalis_B/S. alpinus | **589/524** | TasI | **243/394** | x | x | x | x | x | x |
| Leucaspius delineatus_lineage A | **816** | DraI | **378, 341, 97** | x | x | x | x | x | x |
| Leucaspius delineatus_lineage B | **850** | DraI | **468, 382** | x | x | x | x | x | x |
| Vimba vimba | 593 | VspI | 593 | BlpI | **497, 96** | x | x | x | x |
| Vimba melanops | 594 | VspI | **494, 100** | BlpI | **594** | x | x | x | x |
| V. vimba | **835** | VspI | **835** | AflII | **835** | x | x | x | x |
| V. melanops | **836** | VspI | **597, 239** | AflII | **836** | x | x | x | x |
| Squalius cephalus | **813** | VspI | **460, 353** | AflII | **432, 381** | x | x | x | x |
| genus Vimba/Squalius cephalus | **835/813** | VspI | **835, 597, 460,** 353, 239 | AflII | **835-836, 432, 381** | x | x | x | x |
| Zingel zingel | 441 | MluCI | **363,** 78 | MwoI | 239, **202** | x | x | x | x |
| Zingel streber | 441 | MluCI | **214, 149,** 78 | MwoI | 239, **140, 62** | x | x | x | x |
| Cottus gobio | 423 | BslI | **212,** 125, 86 | PstI | **423** | x | x | x | x |
| Cottus poecilopus | 421 | BslI | 125, **108, 100,** 88 | PstI | **354, 67** | x | x | x | x |
| Pseudorasbora parva_lineage A | **591** | HindII | 266, **175,** 75, 75 | AflII | **478, 89,** 24 | x | x | x | x |
| Pseudorasbora parva_lineage B | **583** | HincII | 264, **243,** 76 | AflII | **529,** 24 | x | x | x | x |
| Neogobius melanostomus | 481 | AluI | **333, 148** | Kpn2I | 481 | DdeI | 481 | x | x |
| Ponticola kessleri | 484 | AluI | 484 | Kpn2I | 484 | Ddel | 484 | x | x |
| Babka gymnotrachelus | 485 | AluI | 485 | Kpn2I | **422, 63** | Ddel | 485 | x | x |
| Neogobius fluviatilis | 482 | AluI | **334, 148** | Kpn2I | 482 | Ddel | **277, 205** | x | x |
| Proterorhinus semilunaris | **465-485** | CseI | 465-485 | DraI | **165-154, 143,** 92, 76 | x | x | x | x |
| Proterorhinus semipellucidus | 483 | CseI | **416, 67** | DraI | **315**, 92, 76 | x | x | x | x |
| Proterorhinus marmoratus | 484 | CseI | 484 | DraI | **392,** 92 | x | x | x | x |
| Gymnocephalus cernua | 602 | EcoT22I | **525,77** | PsiI | 602 | MslI | 405, 197 | x | x |
| Gymnocephalus schraetser | 603 | EcoT22I | 603 | PsiI | **448, 155** | MslI | 400, 203 | x | x |
| Gymnocephalus baloni | 606 | EcoT22I | 606 | PsiI | 606 | MslI | **240, 201, 165** | x | x |
| Lepomis gibbosus | **661** | BseMII | **423, 238** | TaaI | **474,** 187 | DdeI | **194, 171, 129,** 100, 53, 14 | x | x |
| Micropterus salmoides | **641** | BseMII | **439, 202** | TaaI | **310, 188, 143** | DdeI | **332, 160, 96, 53** | x | x |
| Micropterus dolomieu | **638** | BseMII | **638** | TaaI | **312, 145, 135,** 46 | DdeI | **334, 251,** 53 | x | x |
| Barbatula barbatula_lineage A | 436 | SspI | **436** | NhaXI | **390, 46** | x | x | x | x |
| Barbatula barbatula_lineage B | 436 | SspI | **230, 206** | NhaXI | **390, 46** | x | x | x | x |
| Barbatula barbatula_lineage C | 433 | SspI | **230, 203** | NhaXI | **433** | x | x | x | x |
| Sander volgensis | 482 | BalI | **482** | BsuRI | **318,** 144, 20 | x | x | x | x |
| Sander lucioperca | 482 | BalI | **278, 204** | BsuRI | **184,** 144, **134,** 20 | x | x | x | x |
| Perca fluviatilis | **498** | TasI | **443, 55** | Mph1103I | **498** | x | x | x | x |
| Perca flavescens | **483** | TasI | **483** | Mph1103I | **252, 231** | x | x | x | x |
| Cobitis elongatoides | **507** | Mph1103I | **507** | AflII | **359, 148** | x | x | x | x |
| Cobitis strumicae | 517 | Mph1103I | **429, 88** | AflII | 517 | x | x | x | x |
| Cobitis taenia | 516 | Mph1103I | **273, 243** | AflII | 516 | x | x | x | x |
| Cobitis tanaitica | 516 | Mph1103I | **516** | AflII | 516 | x | x | x | x |
| Tinca tinca_lineage Eastern | 656-660 | MboII | **656-660** | TaqI | **656-660** | x | x | x | x |
| Tinca tinca_lineage Western (CZ, 3, 4) | 659 | MboII | **433,** 226 | TaqI | **432, 212,** 15 | x | x | x | x |
| Tinca tinca_lineage Western (1, 2, 5) | 657-659 | MboII | **341,** 226, **90** | TaqI | **432, 212,** 16 | x | x | x | x |
| Hypophthalmichthys molitrix | 487 | RsaI | **337,150** | PfeI | **250,** 192, 45 | x | x | x | x |
| Hypophthalmichthys nobilis | 484 | RsaI | **484** | PfeI | **295,** 189 | x | x | x | x |
| Ctenopharyngodon idella | 481 | RsaI | **322, 159** | PfeI | **425,** 56 | x | x | x | x |
| Phoxinus lumaireul | 467 | XmnI | **278, 85, 63, 41** | DraI | **267, 200** | PacI | **315, 152** | x | x |
| *Phoxinus* phoxinus_lineage A | 462 | XmnI | **277, 144, 41** | DraI | **462** | PacI | 462 | x | x |
| *Phoxinus* phoxinus_lineage B | 467 | XmnI | **319, 144** | DraI | **267, 200** | PacI | 467 | x | x |
| *Phoxinus* phoxinus_lineage C | 463 | XmnI | **319, 144** | Dral | **463** | PacI | 463 | x | x |
| Romanogobio - white-finned group | **806-817** | BslI | **604-610, 202-207** | AvaII | **806-817** | Xap+I | 710-721, 96 | x | x |
| Romanogobio - kesslerii group | 802-806 | BslI | 802-806 | AvaII | 593-600, 206-209 | XapI | 706-710, 96 | x | x |
| Romanogobio - longbarbel group | 811 | BslI | 811 | AvaII | 602, 209 | XapI | **436, 279, 96** | x | x |
| Romanogobio belingi | 806 | VspI | **417, 389** | DraI | 806 | x | x | x | x |
| Romanogobio vladykovi | 808 | VspI | **808** | DraI | 808 | x | x | x | x |
| Romanogobio albipinnatus | **817** | VspI | **817** | DraI | **469, 348** | x | x | x | x |
| Romanogobio kesslerii | **802** | ScaI | **672, 130** | x | x | x | x | x | x |
| Romanogobio banaticus | **806** | ScaI | **806** | x | x | x | x | x | x |
| Cyprinus carpio | **596** | DraI | **596** | AclI | **596** | x | x | x | x |
| Carassius carassius | **588** | DraI | **444, 144** | AclI | **588** | x | x | x | x |
| Carassius gibelio | **568** | DraI | **568** | AclI | **425,** 143 | x | x | x | x |
| Carassius auratus | **579** | DraI | **579** | AclI | **378,** 139, 62 | x | x | x | x |
| Carassius carassius_lineage A | 588 | BsuRI | **397,** 118, 73 | BstNI | **523, 65** | x | x | x | x |
| Carassius carassius_lineage B | 588 | BsuRI | **459,** 118 | BstNI | 588 | x | x | x | x |
| Misgurnus fossilis | **474** | PstI | **398, 76** | BstZ17I | **474** | x | x | x | x |
| Misgurnus nikolskyi | **485** | PstI | **245, 240** | BstZ17I | **434, 51** | x | x | x | x |
| Alburnoides rossicus | 461 | AflII | **461** | ScaI | **297, 164** | x | x | x | x |
| Alburnoides maculatus | 460 | AflII | **309, 151** | ScaI | **460** | x | x | x | x |
| Alburnoides strymonicus | 459 | PvuII | **323, 136** | Alw26I | 194, 175, 90 | BseDI | 459 | x | x |
| Alburnoides economoui | 463 | PvuII | 463 | Alw26I | **373, 90** | BseDI | 463 | x | x |
| Alburnoides thessalicus | 459 | PvuII | 459 | Alw26I | 194, 175, 90 | BseDI | **324, 135** | x | x |
| Alburnoides fasciatus | 463 | AflII | 463 | Alw26I | **198, 175, 90** | x | x | x | x |
| Alburnoides kubanicus | 461 | AflII | **310, 151** | Alw26I | **371, 90** | x | x | x | x |
| Alburnoides eichwaldii | 466 | AflII | 466 | Alw26I | **196, 175, 95** | x | x | x | x |
| Alburnoides sp. 5 | 462 | AflII | 462 | Alw26I | **367, 95** | x | x | x | x |
| Acipenser sturio | **439** | PscI | 439 | DdeI | **203, 136**, 77, 23 | BfaI | 284, 155 | x | x |
| Acipenser ruthenus | **427** | PscI | **247, 180** | DdeI | **187, 164, 76** | BfaI | 282, 145 | x | x |
| Acipenser stellatus | **449** | PscI | 449 | DdeI | **246, 203** | BfaI | **294,** 155 | x | x |
| Acipenser baeri | **427** | PscI | **247, 180** | DdeI | **187, 164, 76** | BfaI | 282, 145 | x | x |
| Acipenser gueldenstaedtii | **443** | PscI | 443 | DdeI | **187, 164, 76** | BfaI | 288, 155 | x | x |
| Huso huso | **443** | PscI | 443 | DdeI | **187, 164, 76** | BfaI | **443** | x | x |
| Bester (H. huso/A. ruthenus) | **443/427** | PscI | **443/247/180** | x | x | BfaI | **443/282/145** | x | x |
| Leuciscus leuciscus | **833** | AluI | **599**, 234 | HindIII | **833** | Bsp143I | **833** | x | x |
| Leuciscus idus | **837** | AluI | **517**, 234, **86** | HindIII | **749, 88** | Bsp143I | **837** | x | x |
| Leuciscus aspius | **835** | AluI | **835** | HindIII | **835** | Bsp143I | **704, 131** | x | x |
| Squalius cephalus | **813** | AluI | **507**, 234, **72** | HindIII | **813** | Bsp143I | **813** | x | x |
| Leuciscus leuciscus_lineage A | 832 | HincII | **399, 348, 92** | BspTI | **832** | x | x | x | x |
| Leuciscus leuciscus_lineage B | 833 | HincII | **833** | BspTI | **682, 151** | x | x | x | x |
| Squalius cephalus_lineage A | 466 | HincII | **386, 80** | DdeI | 251, **150, 49**, 11, 5 | x | x | x | x |
| Squalius cephalus_lineage B | 468 | HincII | **468** | DdeI | 251, **212**, 5 | x | x | x | x |
| Alburnus alburnus_lineage A | **837** | CseI | 837 | SspI | **837** | TaqI | 466, **198**, 173 | x | x |
| Alburnus alburnus_lineage B | **822** | CseI | **461, 361** | SspI | **638, 184** | TaqI | **452,** 197, 173 | x | x |
| Alburnus alburnus_lineage C | **836** | CseI | 836 | SspI | **652, 184** | TaqI | 466, **177**, 173, 20 | x | x |
| A. alburnus/S. cephalus | 471/467 | SsiI | **356, 115/184, 168, 115** | BslI | **316, 155/466** | HhaI | **309, 162/467** | x | x |
| Cyprinidae_hybrids_I Abramis brama | 472 | AanI | **406**, 66 | AluI | **238**, 234 | HincII | **472** | Bsu15I | **472** |
| Scardinius erythrophthalmus | 472 | AanI | **406**, 66 | AluI | **238**, 234 | HincII | **472** | Bsu15I | **371, 101** |
| Ballerus ballerus | **460** | AanI | **262**, 68, 66, 64 | AluI | 234,156,70 | HincII | **460** | Bsu15I | **460** |
| Ballerus sapa | 466 | AanI | **333**, 66, 64 | AluI | 234, **155**, 75 | HincII | **466** | Bsu15I | **466** |
| Rutilus rutilus | 464 | AanI | **333**, 66, 64 | AluI | 234, **155**, 75 | HincII | **389**, 75 | Bsu15I | **464** |
| Blicca bjoerkna | 463 | AanI | **333**, 66, 64 | AluI | **202, 185**, 44, 32 | HincII | **463** | Bsu15I | **463** |
| Cyprinidae_hybrids_II | | | | | | | | | |
| Chondrostoma nasus | 470 | VspI | 358, 112 | TaqI | **366,** 104 | x | x | x | x |
| Rutilus rutilus | 464 | VspI | 355, 109 | TaqI | **190,** 173, 101 | x | x | x | x |
| Blicca bjoerkna | 463 | VspI | **463** | TaqI | **189,** 173, 101 | x | x | x | x |
| Squalius cephalus | 466 | VspI | 352, 114 | TaqI | **293,** 173 | x | x | x | x |
| Cyprinidae_hybrids_III | | | | | | | | | |
| Vimba vimba | **835** | BslI | **553, 282** | BspTI | **835** | x | x | x | x |
| Scardinius erythrophthalmus | **831** | BslI | **707, 124** | BspTI | **733, 98 522, 302** a **518,** | x | x | x | x |
| Leucaspius delineatus - 2 lineages | **824, 790** | BslI | **824, 790** | BspTI | **272** | x | x | x | x |
| Squalius cephalus | **813** | BslI | **600, 213** | BspTI | **430, 383** | x | x | x | x |
| Cyprinidae_hybrids _IV | | | | | | | | | |
| Alburnus alburnus - 3 lineages | 836 | TaiI | **397, 347/333, 92, 15** | AclI | **486, 350/336, 15** | AluI | **522, 234, 80** | x | x |
| Blicca bjoerkna | 833 | TaiI | **741, 92** | AclI | 833 | AluI | **414, 202, 185, 32** | x | x |
| Vimba vimba | 835 | TaiI | **743, 77, 15** | AclI | 835 | AluI | **416, 234, 185** | x | x |
| Abramis brama | 830 | TaiI | **518, 220, 9** | AclI | **521, 309** | AluI | **596, 234** | x | x |
| Leuciscus idus | 837 | TaiI | **513, 232, 92** | AclI | 837 | AluI | **517, 234, 86** | x | x |
| Leuciscus leuciscus | 833 | TaiI | **511, 230, 92** | AclI | 833 | AluI | **601, 232** | x | x |
| Scardinius erythrophthalmus | 472 | AflIII | **444, 28** | Bsp143I | 472 | RsaI | **285**, 98, **66**, 23 | x | x |
| Scardinius hesperidicus | 472 | AflIII | 472 | Bsp143I | **397, 75** | RsaI | **285,** 98**, 66,** 23 | x | x |
| Scardinius acarnanicus | 472 | AflIII | 472 | Bsp143I | 472 | RsaI | **351,** 98, 23 | x | x |

**Table 3. RE assays specific for individual genera and their restriction motifs**

| Genus | RE assay 1 | Restriction motif | RE assay 2 | Restriction motif | RE assay 3 | Restriction motif | RE assay 4 | Restriction motif |
|---|---|---|---|---|---|---|---|---|
| Salvelinus | TasI | AATT | | | | | | |
| Leucaspius | DraI | TTTAAA | | | | | | |
| Vimba | AseI | ATTAAT | BlpI | GCTNAGC | | | | |
| Squalius | AseI | ATTAAT | AflII | CTTAAG | | | | |
| Zingel | TasI | AATT | MwoI | GCNNNNNNNGC | | | | |
| Cottus | BslI | CCNNNNNNNGG | PstI | CTGCAG | | | | |
| Pseudorasbora | HincII | GTYRAC | AflII | CTTAAG | | | | |
| Neogobius and Babka | AluI | AGCT | Kpn2I | TCCGGA | Ddel | CTNAG | | |
| Ponticola | AluI | AGCT | Kpn2I | TCCGGA | Ddel | CTNAG | | |
| Proterorhinus | CseI | GACGCNNNNN | DraI | TTTAAA | | | | |
| Gymnocephalus | EcoT22I | ATGCAT | PsiI | TTATAA | MslI | CAYNNNNRTG | | |
| Lepomis | BseMII | CTCAG(N)10 | TaaI | ACNGT | Ddel | CTNAG | | |
| Micropterus | BseMII | CTCAG(N)10 | TaaI | ACNGT | Ddel | CTNAG | | |
| Barbatula | SspI | AATATT | NhaXI | CAAGRAG | | | | |
| Sander | BalI | TGGCCA | BsuRI | GGCC | | | | |
| Perca | TasI | AATT | EcoT22I | ATGCAT | | | | |
| Cobitis | EcoT22I | ATGCAT | AflII | CTTAAG | | | | |
| Tinca tinca | MboII | GAAGA(N)8 | TaqI | TCGA | | | | |
| Hypophthalmichthys | RsaI | GTAC | PfeI | GAWTC | | | | |
| Ctenopharyngodon | RsaI | GTAC | PfeI | GAWTC | | | | |
| Phoxinus | XmnI | GAANNNNTTC | DraI | TTTAAA | PacI | TTAATTAA | | |
| Romanogobio - differentiation into 3 groups | BslI | CCNNNNNNNGG | AvalI | GGWCC | XapI | RAATTY | | |
| Romanogobio (bel-vla-alb) | AseI | ATTAAT | DraI | TTTAAA | | | | |
| Romanogobio (kess-ban) | ScaI | AGTACT | | | | | | |
| Cyprinus | DraI | TTTAAA | AclI | AACGTT | | | | |
| Carassius (car-gib-aur) | DraI | TTTAAA | AclI | AACGTT | | | | |
| Carassius carassius_2 lineages | BsuRI | GGCC | BstNI | CCWGG | | | | |
| Misgurnus | PstI | CTGCAG | BstZ17I | GTATAC | | | | |
| Alburnoides (Russian-Ukrainian lineages) | AflII | CTTAAG | ScaI | AGTACT | | | | |
| Alburnoides (Aegean lineages) | PvulI | CAGCTG | Alw26I | GTCTCN | BseDI | CCNNGG | | |
| Alburnoides (Ponto-Caspian lineages) | AflII | CTTAAG | Alw26I | GTCTCN | | | | |
| Acipenser | PscI | ACATGT | DdeI | CTNAG | BfaI | CTAG | | |
| Huso | PscI | ACATGT | DdeI | CTNAG | BfaI | CTAG | | |
| Leuciscus | AluI | AGCT | HindIII | AAGCTT | Bsp143I | GATC | | |
| Leuciscus (Aspius) | AluI | AGCT | HindIII | AAGCTT | Bsp143I | GATC | | |
| Squalius | AluI | AGCT | HindIII | AAGCTT | Bsp143I | GATC | | |
| Leuciscus leuciscus - 2 lineages | HincII | GTYRAC | AflII | CTTAAG | | | | |
| Squalius cephalus - 2 lineages | HincII | GTYRAC | DdeI | CTNAG | | | | |
| Alburnus alburnus - 3 lineages | CseI | GACGCNNNNN | SspI | AATATT | TaqI | TCGA | | |
| A. alburnus/S. cephalus hybrid | SsiI | CCGC | BslI | CCNNNNNNNGG | HhaI | GCGC | | |
| Cyprinidae_hybrids_I | | | | | | | | |

| Abramis | AanI | TTATAA | AluI | AGCT | HincII | GTYRAC | Bsu15I | ATCGAT |
|---|---|---|---|---|---|---|---|---|
| Scardinius | AanI | TTATAA | AluI | AGCT | HincII | GTYRAC | Bsu15I | ATCGAT |
| Ballerus | AanI | TTATAA | AluI | AGCT | HincII | GTYRAC | Bsu15I | ATCGAT |
| Rutilus | AanI | TTATAA | AluI | AGCT | HincII | GTYRAC | Bsu15I | ATCGAT |
| Blicca | AanI | TTATAA | AluI | AGCT | HincII | GTYRAC | Bsu15I | ATCGAT |

| Cyprinidae_hybrids_II | | | | | | | | |
|---|---|---|---|---|---|---|---|---|
| Chondrostoma | VspI | ATTAAT | TaqI | TCGA | | | | |
| Rutilus | VspI | ATTAAT | TaqI | TCGA | | | | |
| Blicca | VspI | ATTAAT | TaqI | TCGA | | | | |
| Squalius | VspI | ATTAAT | TaqI | TCGA | | | | |

| Cyprinidae_hybrids _III | | | | | | | | |
|---|---|---|---|---|---|---|---|---|
| Vimba | BslI | CCNNNNNNNGG | AflII | CTTAAG | | | | |
| Scardinius | BslI | CCNNNNNNNGG | AflII | CTTAAG | | | | |
| Leucaspius | BslI | CCNNNNNNNGG | AflII | CTTAAG | | | | |
| Squalius | BslI | CCNNNNNNNGG | AflII | CTTAAG | | | | |

| Cyprinidae_hybrids _IV | | | | | | | | |
|---|---|---|---|---|---|---|---|---|
| Alburnus | TaiI | ACGT | AclI | AACGTT | AluI | AGCT | | |
| Blicca | TaiI | ACGT | AclI | AACGTT | AluI | AGCT | | |
| Vimba | TaiI | ACGT | AclI | AACGTT | AluI | AGCT | | |
| Abramis | TaiI | ACGT | AclI | AACGTT | AluI | AGCT | | |
| Leuciscus | TaiI | ACGT | AclI | AACGTT | AluI | AGCT | | |
| Leuciscus | TaiI | ACGT | AclI | AACGTT | AluI | AGCT | | |
| Scardinius | AflIII | ACRYGT | Bsp143I | GATC | RsaI | GTAC | | |

**Table 4. Species of charrs detectable by the S7iCAPS method**

| English title | Latin title | Origin |
|---|---|---|
| Arctic charr | *Salvelinus alpinus* | Denmark, Germany |
| Brook charr | *Salvelinus fontinalis* | Czech Republic, Slovakia |
| Alsatian char | *Salvelinus alpinus x Salvelinus fontinalis* | Czech Republic |

**Table 5. Species and lineage identification of charrs from the biological material from intensive breeding**

| Results | | Sa | SfA | SfB | SfA/SfB | SalsH2A | SalsH2B |
|---|---|---|---|---|---|---|---|
| Declared | No. | | | | | | |
| Sa | 20 | 20 | | | | | |
| Sf | 42 | | 21 | 4 | 10 | 6 | 1 |
| Sals | 34 | 1 | | | 1 | 22 | 10 |

The method of the invention allows the detection of the respective species/lineages in samples of fresh, frozen, fixed food and in all samples of food, feed and generally products containing fish meat.

Another object of the invention are kits for identification of species/lineages of European freshwater fish using the method according to the invention. Identification kits contain the species-specific primer pairs and specific restriction endonucleases producing species/lineages specific restriction patterns, by means of which determination of homozygous ("pure"), heterozygous and hybrid individuals is carried out in biological materials and fish products. Even simple electrophoretic patterns (without restriction enzyme digestion) visualizing length polymorphism of the obtained amplicons - JGM regions, which are also used in the invention, are in some cases sufficient for identification.

The method of the invention relates to conventional PCR using specific primers, which define species-unique intronic JGM regions and generate either length polymorphic amplicons and/or specific restriction patterns in conjunction with digestion by specific restriction endonucleases designed into indel positions and/or SNPs positions. The method, called S7iCAPS (S7 intron cleaved amplified polymorphic sequence), which is also the object of the invention, was derived from the subsequent conversion of the unique indels and SNPs to the species/lineages specific CAPS markers (cleaved amplified polymorphic sequence). The S7iCAPS method was designed for routine use in the laboratory to identify the above mentioned taxa of European ichthyofauna. It is based on the amplification of partial sequences of the first intron of S7 r-protein (S7).

S7iCAPS identification method utilizes a combination of: 1) a diagnostic length of the PCR products (due to the presence of sufficiently long indels) and specific SNP-CAPS or indel CAPS marker, or 2) two InDel-CAPS or SNP-CAPS markers or combinations thereof.

New primer design for European species or groups of species targeted to conservative regions and delimiting the hypervariable species/lineages specific parts of S7 intronic sequence, the so-called JGM regions, was designed. Optimization for all the components, steps and temperature profiles was also made.

A unique advantage of the method of the invention is that the developed arrangement designed on biparental mode of inheritance of S7 alleles and their possible allelic recombination allows to detect historical hybridization events occurring by both sexual and asexual reproduction of fish.

Another advantage is that the temperature profile for all monoplex PCR is universal and differs only in a few cases by the annealing temperature. Also chemical reagents have been tuned to a single mastermix concept. This is advantageous for processing of large numbers of samples, where species/lineages different samples can be analyzed simultaneously in one experiment, which leads to significant cost and time savings.

Another advantage is that the costs of CAPS assays are generally low because cheap and commonly available restriction enzymes are used.

The resulting PCR products are specific and it is not necessary to verify their specificity by other methods. Nevertheless, if necessary, the abovementioned primers can also be used as sequencing primers for a quick and easy confirmation, which is inexpensive because of the application of only one primer pair, i.e. two runs on a conventional genetic analyser.

Another advantage is that the length of the J. G. Mendel diagnostic region is designed in the range from about 450 to 850 bp, which is sufficient to produce an easily recognizable restriction pattern on an agarose gel.

The reliability and robustness of the proposed arrangement were tested on samples from different geographic areas (different Sea drainage areas and basins), as well as on various cyclers and mastermixes from diverse companies.

S7iCAPS identification key is designed for two stages and in some cases even for three stages. All samples can be analyzed by using at least two different restriction enzymes designed into indel and/or SNPs positions. In some cases, one of them can be replaced by diagnostic length polymorphism of amplicons, which provides sufficient reliability of determination.

The main advantage of the S7iCAPS method is the easy repeatability and applicability (possibility to perform in different laboratories with conventional instrumentation without further optimization), time savings and low financial costs compared to the next generation sequencing technologies. The present process does not require the use of commercial reference standards, which are necessary for methods based on protein analysis.

The method of the present invention has a particular importance in the mapping of species richness and real intraspecific (genetic) diversity of ichthyofaunas, in terms of protection of the original gene-pool of fish in different countries, assessment of introduction and reintroduction efforts and as a tool for prevention of devastating threats and health hazards of imports of non-native species/lineages of fish. Furthermore, the method is important in the area of genetic determination of individual lineages and breeds in intensive farming in terms of the breeding management, to preserve the breed purity, to increase and to improve production efficiency, for the preservation of resistance to diseases and various stress factors. Also the area of prevention of falsification of fish eggs, fingerlings, fry material and fish-containing products is considerable, where specific mix-ups with a different quality and price occur.

The following embodiment illustrates the method of the invention, without limiting it in any way.

### Brief description of the drawings

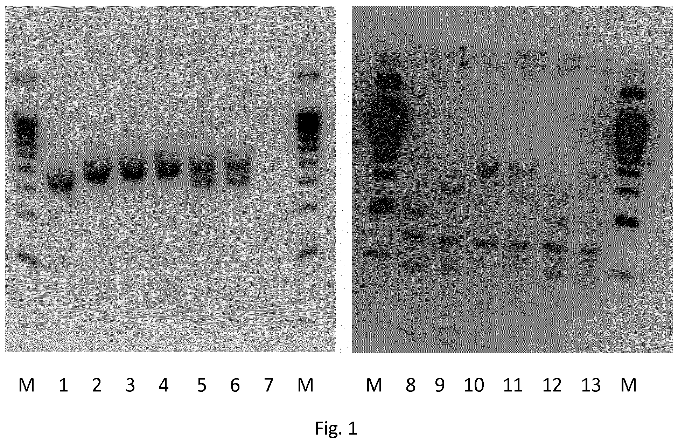

Figure 1 - Specific JGM regions and TasI determination with restriction patterns of the pure species of charrs, their intraspecific heterozygotes and interspecific hybrids.

### 1. Unique JGM region

M = Marker (155-970 bp)
1 = S. alpinus (524 bp)
2 = S. fontinalis variant A (583 bp)
3 = S. fontinalis variant B (589 bp)
4 = heterozygous S. fontinalis A/B (583/589 bp)
5 = hybrid S. alpinus/S. fontinalis variant A (524/583 bp)
6 = hybrid S. alpinus/S. fontinalis variant B (524/589 bp)
7 = negative control
M = Marker (155-970 bp)

### 2. TasI determination (TasI assay)

M = Marker (155-970 bp)
8 = S. alpinus (diagnostic fragment 243 bp)
9 = S. fontinalis variant A (diagnostic fragment 305 bp)
10 = S. fontinalis variant B (diagnostic fragment 394 bp)
11 = heterozygous S. fontinalis A/B (305/394 bp)
12 = hybrid S. alpinus/S. fontinalis variant A (243/305 bp)
13 = hybrid S. alpinus/S. fontinalis variant B (243/394 bp)
M = Marker (155-970 bp)

### Examples

### Example 1

### Preparation of the material and reagents and conventional methodology

DNA material. For verification of the new identification S7iCAPS method in routine analysis, DNA templates obtained from 96 charr samples from two Czech breeding facilities originating from six Czech and foreign suppliers (Table 4) were used. Specifically, the samples were parts of fins retained in ethanol. As a positive control, charr samples with certification from the supplier were used, which were, in addition, morphologically and genetically pre-tested.

Sampling. From each source 10-55 fin tissue samples were taken for PCR analysis.

DNA isolation. The isolation was made using ZR Genomic DNA-Tissue MiniPrep kit (Zymo Research, USA) according to the manufacturer's instructions. The DNA was isolated from 25 mg of tissue. The concentration and purity of the DNA template was measured by Helios γ device (Thermo Spectronic, GB).

Primer design. Primer set for hypervariable region of nuclear sequence of the first intron of the S7 gene (JGM region) was designed by program Oligo (Molecular Biology Insights, USA), Table 1.

Monoplex PCR system. Reagents for monoplex PCR were optimized, including temperature conditions of PCR protocol. Optimized reaction mixture contained 12,5 µl of PPP Master Mix (Top-Bio, Czech Republic; composition: Tris-HCl, (NH₄)₂SO₄, Tween 20, MgCl₂, dATP, dCTP, dGTP, dTTP, Taq-Purple DNA polymerase, stabilizers and additives), 20 pmol of each primer SalS7F and SalS7R and 3 µl of isolated DNA in the total volume 25 µl. PCR amplification proceeded in the thermocycler Mastercycler pro (Eppendorf, Germany) at the following conditions: initial denaturation for 2 min at 95 °C, followed by 30 cycles for 30 s at 95 °C, 45 s at 60 °C, 1 min at 72 °C and, at the end, final elongation for 10 min at 72 °C.

Fragment separation and visualization. PCR amplification products and products of restriction digestion were separated on 1.7% agarose gels by horizontal electrophoresis, using SB buffer and Midori Green Advance staining at 10 V/cm for 45-50 minutes. DNA marker 155-970 (Top-Bio, Czech Republic) was used as a size standard. Individual fragments were visualized by gel documentation and analytical system GeneGenius Match (Trigon-plus, Czech Republic).

### Example 2

### Identification method

Method S7iCAPS. S7iCAPS identification method of charrs utilizes combined "amplicon-SNP-CAPS" design, i.e. the presence of two indels providing different lengths of PCR products of the variable region of the first intron, as well as targeted digestion into species/lineages specific SNP. Two programs, CAPS Designer (http://solgenomics.net/tools/caps_designer/caps_input.pl) and SNP2CAPS (Thiel, T., Kota, R., Grosse, I., Stein, N., Graner, A. 2004. SNP2CAPS: a SNP and INDEL analysis tool for CAPS marker development. Nucleic acid research 32 (1), e5) were used as a tools for CAPS design for computer conversion of SNPs to CAPS markers. Sixty-four CAPS candidates were found, of which TasI was chosen as the most suitable (Table 3).

JGM regions length. The lengths of the amplified PCR products are species/lineages-specific and for *Salvelinus alpinus* (Sa) it is 524 bp, for *Salvelinus fontinalis* lineage "A" (SfA) 583 bp and for *Salvelinus fontinalis* lineage "B" (SfB) 589 bp. In case of Alsatian char detection (interspecific hybrid - *Salvelinus fontinalis* x *Salvelinus alpinus*), the specific lengths for SalsH2A and SalsH2B type hybrid are 524/583 and 524/589, respectively (Table 2).

Corroborative SNP-CAPS marker TasI. PCR amplification product is subsequently digested (according to the manufacturer's instructions) by species/lineages-specific restriction enzyme TasI with restriction site "/AATT" in the region defined by the primer pair. This SNP-CAPS marker (TasI assay) confirms the correct species determination as well as distinguishes more reliably both lineages of *Salvelinus fontinalis* from each other. The length of *Salvelinus alpinus* diagnostic restriction fragment is 243 bp, *Salvelinus fontinalis* lineage "A" 305 bp and lineage "B" 394 bp (Table 2, Figure 1).

A more sensitive resolution of heterozygotes and interspecific hybrids. The resolution power of TasI assay is also used for unambiguous resolution of intraspecific heterozygotes of brook charr, as well as interspecific hybrids of Alsatian char. The specific TasI restriction pattern for distinguishing the intraspecific heterozygote A/B of brook charr is 305/394 and for interspecific hybrid of Alsatian char "A" and "B" type, it is 243/305 and 243/394, respectively.

Verification of heterozygous nuclear haplotypes. For a reliable identification of individual S7 haplotypes, the resulting PCR products were cloned and sequenced. Moreover, all heterozygous genotypes were compared to homozygous haplotypes and confirmed by the Phase method implemented in program DnaSP (Librado, P., Rozas, J. 2009. DNASP v5: A software for comprehensive analysis of DNA polymorphism data. Bioinformatics, 25, 1451-1452).

Interlaboratory test. The reliability of the S7iCAPS method was tested based on interlaboratory tests of unspecified samples. The samples were sent for an analysis by cloning and subsequent sequencing to the university lab. The same blind samples were analyzed by sequencing in a commercial laboratory.

Prevention of contamination. The PCR method is characterized by a high sensitivity, it is therefore necessary to follow strict rules of a good laboratory practice. DNA extraction and mastermix preparation with the addition of the template were spatially separated. All tools and pipettes were sterilized prior to use in the UV crosslinker BLX-254 (Vilber Lourmat, France) and all plastic material was sterilized in a steam sterilizer Vaposteri/P (BMT, Czech Republic). The tips with filters were used for handling. At each amplification, a negative control sample (PCR H₂O) was used to exclude contamination of the reagents and plastics.

### Example 3

### Verification of the reliability of the S7iCAPS method on commercial samples

Reliability of the primer design. The designed primers were tested for reliable PCR amplification of individual taxa, as well as for their suitability for sequence analysis. It was laboratory verified that the primer pair amplified and sequenced the analyzed species, including hybrids, correctly.

Specificity of the JGM regions lengths. Specific lengths of the unique JGM regions of the examined taxa were analyzed and their diagnostic character was clearly demonstrated (Figure 1).

Specificity of TasI assay. Specificity of TasI assay and feasibility conditions proposed by the producer were tested. Diagnostic character of restriction patterns and functionality of the proposed conditions were demonstrated (Figure 1).

Investigation results. S7iCAPS method was tested for two commercial farms with analysis of 96 samples from various domestic and foreign suppliers.

Out of 96 samples tested, 42 samples were declared as pure brook charr without further lineage splitting and in 35 samples (83 %) it was confirmed. In addition also detailed classification into two different lineages "A" and "B" was done. Lineage A was distinguished in 21 samples, lineage B in 4 samples and interlineage heterozygote (A/B) was detected in 10 samples. In 7 samples (17 %), a completely different species of charr was detected, Alsatian char, which originates by targeted hybridisation of arctic charr with brook charr. Twenty samples were declared as the arctic charr from two different sources and all twenty (100 %) were confirmed as species belonging to the arctic charr, but only from one source. Thirty-four samples were identified as Alsatian char, with 32 samples (94 %) confirmed. Additionally, a detailed classification of Alsatian char revealed 22 hybrids with brook charr lineage "A" (H2A) participation and 10 hybrids with brook charr lineage "B" (H2B) participation. In two samples (6 %), a completely different species of charr was detected, pure arctic charr in one case and intraspecific heterozygote A/B of brook charr in the second case (Table 5).

The reliability of the developed method of identification according to the invention was tested by analyzing blank samples prepared by the authors. Reference samples were analyzed by cloning and sequencing methods in the university lab. The same blind samples were analyzed by a foreign commercial laboratory by direct sequencing method. The obtained results were compared with the results that the inventors obtained and no difference was found.

### Industrial applicability

The identification method of European ichthyofauna taxa is intended for routine genetic identification of freshwater fish and their hybrids in biological materials and fish products. An important prerequisite for the successful use is the isolation of a high quality DNA and proper treatment by the selected restriction endonucleases according to the manufacturer's instructions. The developed S7iCAPS method was tested in practice for the samples of the mentioned genera and species from different geographical areas. The method includes conventional PCR methodology, which includes PCR mastermix composition with proportion of individual components, PCR temperature protocol and analysis of individual restriction patterns or length polymorphism of diagnostic JGM regions. The proposed configuration is useful for both public and private genetic laboratories, research institutes, fish breeding facilities (fish farming, fishery, etc.), basin authorities, fishing associations and local organizations, agencies for conservation of nature and individual PLA administrations, universities of agricultural and natural science, food and customs inspection, the Ministry with focus on agriculture and environment, national museums, etc., both within and outside the Czech Republic.

This method includes S7iCAPS methodology that includes the composition of the PCR mastermix with specific primer pairs and proportion of other components, temperature-specific PCR protocol and analysis of results of species/lineages by specific CAPS markers or unique amplicon lengths, if they are determined.

## Claims

1. A method of identification of freshwater European fish in biological materials and fish products by S7iCAPS method **characterized by** that a DNA sample from a biological material or a fish product is subjected to a PCR analysis using species-specific or group-specific primer pairs presented in Table 1 which define a partial species/lineage-specific sequence of the first intron of S7 gene, the so-called J. G. Mendel diagnostic region (JGM), wherein the diagnostic JGM regions are specifically defined for each individual species and have its own specific length presented in Table 2, whereupon, based on the length of the PCR products, identification of the species or hybrid is carried out.

2. The method of identification according to claim 1, **characterized by** that the DNA sample is inserted into a mixture of PPP Master Mix consisting of Tris-HCl, (NH₄)₂SO₄, Tween 20, MgCl₂, dATP, dCTP, dGTP, dTTP, Taq-Purple DNA polymerase, stabilizers and additives, and at least one specific primer pair, in which the initial denaturation is carried out for 2 minutes at the temperature of 95 °C, followed by 30 cycles for 30 s at 95 °C, 45 s at 60 °C or at the annealing temperature presented in Table 1, 1 minute at 72 °C and, finally, the final elongation is carried out for 10 minutes at 72 °C.

3. The method according to claim 1 or 2, **characterized by** that after the PCR analysis, the PCR products are digested by species/lineage-specific restriction endonucleases in a specific restriction site which is located in the diagnostic JGM region in order to confirm the specific length of the non-digested JGM region or in order to distinguish among the species having a similar JGM region length or various species lineages and interspecific hybrids, wherein the specific restriction endonucleases and the restriction sites or motifs for individual species are presented in Tables 2 and 3, whereupon an identification of the species, intraspecific heterozygote or interspecific or intergeneric hybrid is carried out based on the combination of the lengths of the specifically digested fragments.

4. The method according any of claims 1 to 3, **characterized by** that the DNA sample is obtained from samples of fresh, frozen, fixed biological materials or from samples of food, feed and generally products containing fish meat.

5. An identification kit for species/lineage identification of freshwater European fish by the method defined in any of claims 1 to 4, **characterized by** that it comprises species-specific primer pairs presented in Table 1, a protocol of PCR reaction conditions including the specific temperatures and cycle times, description of the method of identification and interpretation, including a list of JGM lengths, a list of the specific restriction endonucleases with a list of the assigned restriction motifs and specific lengths of the digested fragments.

6. The identification kit according to claim 5, **characterized by** that it further comprises PPP Master Mix consisting of Tris-HCl, (NH₄)₂SO₄, Tween 20, MgCl₂, dATP, dCTP, dGTP, dTTP, Taq-Purple DNA polymerase, stabilizers and additives,.

7. The identification kit according to claim 5 or 6, **characterized by** that it further comprises the restriction endonucleases presented in Table 3.

## Patentansprüche

1. Verfahren zur Identifizierung europäischer Süßwasserfische in biologischen Materialien sowie in Fischprodukten unter Verwendung der S7iCAPS-Methode, **dadurch gekennzeichnet, dass** eine aus einem biologischen Material oder einem Fischprodukt erhaltene DNA-Probe einer PCR-Analyse unter Verwendung der in der Tabelle 1 aufgelisteten gattungs- oder gruppenspezifischen Primerpaare unterzogen wird, welche Primerpaare die partielle gattungs-/linienspezifischen Sequenz des ersten Introns des S7-Gens abgrenzen, die als diagnostische Gebiet nach J. G. Mendel (JGM) bezeichnet wird, wobei die diagnostischen Gebiete nach JGM namentlich für eine bestimmte Spezies abgegrenzt sind und ihre spezifische, in der Tabelle 2 aufgelistete Länge aufweisen, und anschließend aufgrund der Länge der PCR-Produkte die Identifizierung der Spezies oder eines Hybriden derselben durchgeführt wird.

2. Verfahren zur Identifizierung nach Anspruch 1, **dadurch gekennzeichnet, dass** die DNA-Probe in ein Gemisch von PPP Master Mix eingeführt wird, das aus Tris-HCl, (NH₄)₂SO₄, Tween 20, MgCl₂, dATP, dCTP, dGTP, dTTP, Taq-Purple DNA-Polymerase, Stabilisatoren sowie Zusatzstoffen besteht und wenigstens ein spezifisches Paar von Primeren enthält, in welchem Gemisch die anfängliche, 2 Minuten lang bei der Temperatur von 95 °C verlaufende Denaturierung erfolgt, anschließend 30 Zyklen von 30 s bei 95 °C, 45 bei 60 °C oder bei einer der in der Tabelle 1 angeführten Annealingtemperaturen, 1 Minute bei 72 °C durchgeführt werden und abschließend die endgültige Dehnung erfolgt, die 10 Minuten lang bei 72 °C verläuft.

3. Verfahren nach Anspruch 1 oder 2, **dadurch gekennzeichnet, dass** die PCR-Produkte im Anschluss an die PCR-Analyse mithilfe von gattungs-/linienspezifischen Restriktionsendonukleasen in einer innerhalb des diagnostischen Gebiets nach JGM befindlichen Restriktionsstelle zwecks der Bestätigung der spezifischen Länge des nicht spaltenden Gebiets nach JGM und/oder zwecks der Differenzierung der Spezies mit ähnlichen Längen des Gebiets nach JGM oder der Differenzierung von unterschiedlichen Gattungslinien oder Interspezies-Hybriden gespalten werden, wobei die spezifischen Restriktionsendonukleasen sowie Restriktionsstellen oder Motive in den Tabellen 2 und 3 aufgelistet sind, und anschließend aufgrund der Kombination der Längen der spezifisch verdauten Fragmente die Identifizierung der Spezies, des Intraspezies-Heterozygoten oder des Interspezies- Hybriden, bzw. des intergenerischen Hybriden durchgeführt wird.

4. Verfahren nach einem der Ansprüche 1 bis 3, **dadurch gekennzeichnet, dass** die DNA-Probe aus Proben erhalten wird, die frische, gefrorene oder fixierte biologische Materialien, bzw. Nahrungsmittel, Futtermittel und, im Allgemeinen, Fischfleisch enthaltende Produkte umfassen.

5. Kit zur gattungs-/liniengemässen Identifizierung europäischer Süßwasserfische mittels des Verfahrens nach einem der Ansprüche 1 bis 4, **dadurch gekennzeichnet, dass** der Kit die in der Tabelle 1 aufgelisteten gattungsspezifischen Primerpaare, ein PCR-Protokoll von Reaktionsbedingungen mit spezifischen Temperaturen und Zykluslängen, eine Beschreibung des Verfahrens zur Identifizierung und Auswertung einschließlich der Auflistung der Längen der Gebiete nach JGM, ein Verzeichnis von spezifischen Restriktionsendonukleasen einschließlich der Auflistung der zugeordneten Restriktionsmotiven und der spezifischen Längen der aufgespaltenen Fragmente umfasst.

6. Kit zur Identifizierung nach Anspruch 5, **dadurch gekennzeichnet, dass** der Kit ferner ein Gemisch von PPP Master Mix umfasst, dass aus Tris-HCl, (NH₄)₂SO₄, Tween 20, MgCl₂, dATP, dCTP, dGTP, dTTP, Taq-Purple DNA-Polymerase, Stabilisatoren und Zusatzstoffen besteht.

7. Kit zur Identifizierung europäischer nach Anspruch 5 oder 6, **dadurch gekennzeichnet, dass** der Kit ferner die in der Tabelle 3 aufgelisteten Restriktionsendonukleasen umfasst.

## Revendications

1. Un procédé d'identification des poissons d'eau douce européens dans les matières biologiques et dans les produits du poisson par la méthode S7iCAPS, **caractérisé en ce que** un échantillon d'ADN provenant d'une matière biologique ou d'un produit du poisson est soumis à une analyse PCR, en utilisant les paires d'amorces spécifiques pour une espèce ou pour un groupe présentées au Tableau 1, cettes paires définissant la séquence partielle spécifique pour les espèces/lignages du premier intron du gène S7, qui s'apelle la zone de diagnostic de J. G. Mendel (JGM), où les zones de diagnostic JGM sont spécifiquement définies pour chaque espèce et ont leur propre longueur présentée au Tableau 2, après quoi l'identification de l'espèce ou de l'hybride est effectuée basée sur la longueur des produits PCR.

2. Le procédé d'identification selon la revendication 1, **caractérisé en ce que** échantillon d'ADN l'échantillon d'ADN est inséré dans un mélange de PPP Master Mix composé de Tris-HCl, (NH₄)₂SO₄, Tween 20, MgCl₂, dATP, dCTP, dGTP, dTTP, polymérase ADN Taq-Purple, stabilisants et additifs, et au moins une paire d'amorces spécifique, où la dénaturation initiale est effectuée pendant 2 minutes à la température de 95 °C, suivie de 30 cycles pendant 30 secondes à 95 °C, 45 secondes à 60 °C ou à la température d'annelage présentée au Tableau 1, 1 minute à 72 °C et, finalement, l'allongement final est effectué pendant 10 minutes à 72 °C.

3. Le procédé selon la revendication 1 ou 2, **caractérisé en ce qu'**après l'analyse du PCR, les produits du PCR sont digérés par endonucléases de restriction spécifiques pour l'espèces/lignages dans un site de restriction particulier qui se trouve dans la zone de diagnostic de JGM afin de confirmer la longueur spécifique de la zone de JGM non-digérée ou afin de distinguer entre les espèces ayant une longueur semblable de la zone JGM ou des lignées d'espèces diverses et des hybrides interspécifiques, où les endonucléases de restriction spécifiques et les sites ou motifs de restriction pour chaque espèce sont présentés dans les Tableaux 2 et 3, où l'identification de l'espèce, l'hétérozygote intraspécifique ou l'hybride interspécifique ou intergénérique est effectuée en fonction de la combinaison des longueurs des fragments spécifiquement digérés.

4. Le procédé selon l'une quelconque des revendications 1 à 3, **caractérisé en ce que** l'échantillon d'ADN est obtenu à partir d'échantillons de matières biologiques fraîches, congelées ou fixées ou d'échantillons d'aliments, de fourrage et généralement de produits contenant de la viande de poisson.

5. Une trousse d'identification pour l'identification des espèces/lignages de poissons européens d'eau douce par le procédé défini dans l'une quelconque des revendications 1 à 4, **caractérisé en ce qu'**elle comprend les paires d'amorces spécifiques à des espèces présentées au Tableau 1, un protocole des conditions de réaction de PCR, y compris les températures et les temps des cycles spécifiques, une description du procédé d'identification et d'interprétation, y compris une liste des longueurs des zones JGM, une liste des endonucléases de restriction spécifiques avec une liste des motifs de restriction assignés et des longueurs spécifiques des fragments digérés.

6. La trousse d'identification selon la revendication 5, **caractérisé en ce qu'**elle comprend également le PPP Master Mix composé de Tris-HCl, (NH₄)₂SO₄, Tween 20, MgCl₂, dATP, dCTP, dGTP, dTTP, polymérase ADN Taq-Purple, stabilisants et additifs.

7. La trousse d'identification selon la revendication 5 ou 6, **caractérisé en ce qu'**elle comprend également les endonucléases de restriction présentés dans le Tableau 3.
